(19) Europäisches Patentamt
European Patent Office
Office européen des brevets

(11) Veröffentlichungsnummer: **0 016 420**
**B1**

(12) **EUROPÄISCHE PATENTSCHRIFT**

(45) Veröffentlichungstag der Patentschrift: **27.01.82**

(51) Int. Cl.³: **C 07 D 277/16**

(21) Anmeldenummer: **80101290.7**

(22) Anmeldetag: **13.03.80**

(54) **Verfahren zur Herstellung von Thiazolidin-2-thionen.**

(30) Priorität: **24.03.79 DE 2911661**

(43) Veröffentlichungstag der Anmeldung: **01.10.80 Patentblatt 80/20**

(45) Bekanntmachung des Hinweises auf die Patenterteilung: **27.01.82 Patentblatt 82/4**

(84) Benannte Vertragsstaaten: **BE CH DE FR GB IT NL**

(56) Entgegenhaltungen:
**US-A-3 215 703**
**US-A-3 215 704**
**JOURNAL OF THE CHEMICAL SOCIETY, 1949, Teil II, The Chemical Society, Seiten 786—789, London, G.B.**
**J. W. BATTY et al.: »Acetylene Reactions. Part III. Reaction of Aminobutynes with Carbon Disulphide«, Seite 789**
**Ullmanns Encyklopädie der technischen Chemie, 4. Auflage, Band 1, Seiten 227—8 und 285—6.**

(73) Patentinhaber: **BAYER AG, Zentralbereich Patente, Marken und Lizenzen, D-5090 Leverkusen 1, Bayerwerk (DE)**

(72) Erfinder: **Hüllstrung, Dieter, Dr., Friedrich-Bayer-Strasse 11, D-5090 Leverkusen 1 (DE)**
Erfinder: **Trimbach, Jürgen, Roggendorfstrasse 103, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 016 420 B1

BUNDESDRUCKEREI BERLIN

## Verfahren zur Herstellung von Thiazolidin-2-thionen

Die Erfindung betrifft ein Verfahren zur Herstellung von Thiazolidin-2-thionen durch Umsetzung von Aminoalkoholen mit Schwefelkohlenstoff.

Aus J. Chem. Soc., 1949, 789, ist es bekannt, N-substituierte Thiazolidin-2-thione aus den entsprechenden N-substituierten Aminoethanolen und Schwefelkohlenstoff bei 120 – 140° C und 2 – 5 Stunden Verweilzeit in einer einstufigen Druckreaktion im Autoklaven herzustellen. Die Ausbeute wird mit 65 – 75% angegeben. Die US-PS 3 215 703 sagt aus, daß diese Produkte wegen großer Mengen an gefärbten und übelriechenden Nebenprodukten umkristallisiert werden müssen, bevor sie als Vulkanisationsbeschleuniger für Kautschuke verwendet werden können. Ein verbessertes Verfahren gemäß US-PS 3 215 704 wird bei <75 – 160° C und ca. 6 Stunden Verweilzeit in einer drucklosen Zweistufenreaktion in einem Rührkessel mit Rückflußkühler durchgeführt. Die Ausbeute ist wesentlich höher. Jedoch birgt dieses Verfahren bei der technischen Durchführung Schwierigkeiten und Risiken, u. a. wegen des Auftretens schwefelkohlenstoffhaltiger Abgase gegen Ende der diskontinuierlich ablaufenden Reaktion, die eine sorgfältige Überwachung der einzelnen Reaktionsschritte notwendig machen. Das Verfahren ist daneben auch wegen der langen Reaktionszeit von 6 Stunden noch unbefriedigend.

Es wurde nun gefunden, daß man Thiazolidin-2-thione bei einem Druck von 0,1 – 5 bar und 20 – 200° C in ausgezeichneter Ausbeute aus 1 Mol des entsprechenden Aminoäthanols und 2 bis 2,1 Mol des Schwefelkohlenstoffs herstellen kann, indem in einem kontinuierlichen Einstufenprozeß in mindestens zwei hintereinandergeschalteten Reaktionszonen die Flüssigphase, die das Aminoäthanol enthält, in innige Berührung mit der Gasphase, die Schwefelkohlenstoff enthält, gebracht wird, wobei die besagte Flüssigphase kontinuierlich in die erste Reaktionszone eingebracht, in Richtung bis zur letzten Zone geführt und hinter der letzten Zone entnommen wird, während gleichzeitig die besagte Gasphase kontinuierlich in die letzte Reaktionszone eingebracht, in Richtung bis zur ersten Zone geführt und hinter der ersten Zone entnommen wird.

Als Thiazolidin-2-thione seien Verbindungen der Formeln (1) und (2) genannt:

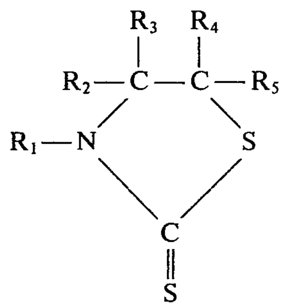

(1)

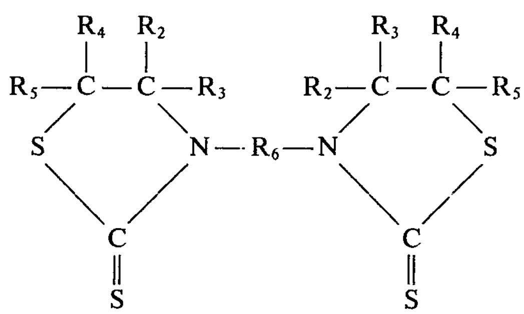

(2)

in denen

$R_1$ Wasserstoff, einen geradkettigen, verzweigten oder cyclischen Alkyl- oder Alkenylrest, dessen C-Kette gegebenenfalls durch N, O oder S unterbrochen ist und der weiterhin gegebenenfalls durch ein oder mehrere Aryl-, Hydroxyl- und/oder Halogengruppen substituiert ist, bedeutet,
$R_2 - R_5$ gleich oder verschieden Wasserstoff, Phenyl, Alkyl, darstellen,
$R_2$ und $R_4$ oder $R_3$ und $R_5$ gemeinsam eine Polymethylenbrücke mit 2 bis 6 C-Atomen bilden,
$R_6$ eine Alkylen-, Cycloalkylen- oder Xylylengruppe bedeutet.

Der Substituent $R_1$ bedeutet besonders bevorzugt $C_1 - C_{18}$-Alkyl, insbesondere $C_1 - C_8$-Alkyl, $C_3 - C_{18}$-Alkenyl, insbesondere $C_3 - C_8$-Alkenyl, das bevorzugt einfach durch eine $C_6 - C_{12}$-Arylgruppe, eine Hydroxygruppe oder durch Halogen wie Chlor, Brom, Jod, Fluor substituiert sein kann.

In den Substituenten $R_2-R_5$ bedeutet Alkyl bevorzugt $C_1-C_{18}$-Alkyl, insbesondere $C_1-C_8$-Alkyl. Diese Alkylgruppe kann gegebenenfalls durch ein oder mehrere, bevorzugt eine Halogengruppe (Chlor, Brom, Jod, Fluor), OH-Gruppe, COOH-Gruppe oder $COOR_7$-Gruppe mit $R_7$ gleich $C_1-C_5$-Alkyl substituiert sein.

Der Substituent $R_6$ stellt bevorzugt $C_2-C_6$-Alkylen, $C_5-C_6$-Cycloalkylen oder eine Xylylengruppe dar.

Die als Ausgangsmaterial eingesetzten Aminoalkohole stellen Verbindungen mit bevorzugt folgenden Formeln (3) und (4) dar:

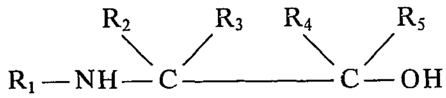 (3)

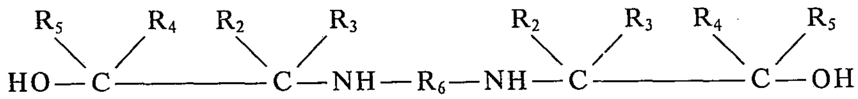 (4)

in denen
$R_1$ bis $R_6$ die bereits angegebene Bedeutung besitzen.

Unter Reaktionszonen im obengenannten Sinne sind Behälter zu verstehen, die miteinander durch beispielsweise Rohre, Fallschächte, Spalte, Siebplatten oder Lochplatten oder aber ohne jegliche Abgrenzung verbunden sind, wenn sie die Erfüllung der Forderung nach inniger Berührung von Gasphase und Flüssigphase gewährleisten, wie beispielsweise Kessel mit Zu- und Ablaufrohren für die Gas- und Flüssigphase, mit Rührwerk versehene Kessel dieser Art, Glockenboden-, Siebboden-, Kreuzstrombodenstufen nach der Bauweise von Destillationskolonnen oder mit Füllkörpern versehene Abschnitte einer Füllkörperkolonne, die ohne erkennbare obere und untere Abgrenzung vom Fachmann als »Boden« oder »Stufe« bezeichnet werden. Es können selbstverständlich auch verschieden gestaltete derartige Reaktionszonen zu der Apparatur zusammengesetzt werden, in der das erfindungsgemäße Verfahren durchgeführt wird, beispielsweise Rührkessel und Füllkörperabschnitt oder Glockenbodenstufen und Füllkörperabschnitte.

Die Reaktionszonen werden im allgemeinen weder beheizt noch gekühlt. Die einzelnen Reaktionszonen können aber mit Wärmeaustauschaggregaten ausgerüstet werden, um die Reaktionswärme abzuführen, oder Wärme zuzuführen. Nach einer bevorzugten Ausführungsform des Verfahrens wird jedoch ein Teil der Reaktionswärme durch Verdampfen des in den Reaktionszonen befindlichen Aminoethanols bzw. des gebildeten Thiazolidin-2-thione entnommen, wobei beispielsweise der Gasstrom vor oder hinter der ersten Reaktionszone abgekühlt und die hierbei auskondensierte Flüssigkeit in eine der Reaktionszonen zurückgeführt wird. Das auf diese Weise erhaltene Abgasgemisch ist praktisch frei von Schwefelkohlenstoff.

Ebenso wird ein Teil der Reaktionswärme bevorzugt dadurch abgeführt, daß die Flüssigphase vor oder hinter der letzten Reaktionszone gekühlt wird.

Das hinter der letzten Reaktionszone anfallende flüssige Gemisch enthält neben dem gewünschten Thiazolidin-2-thion noch etwas Schwefelkohlenstoff und gelöste Beiprodukte. Der Schwefelkohlenstoff kann in bekannter Weise durch Einleiten von beispielsweise Stickstoff oder Wasserdampf aus dem Gemisch entfernt werden. Nach einer bevorzugten Ausführungsform wird er jedoch durch Zugabe einer Menge an besagtem Aminoethanol, die mindestens der doppelten molaren Menge des noch vorhandenen freien Schwefelkohlenstoffs entspricht, unter Bildung eines gelösten Beiproduktes vollständig umgesetzt.

Das besagte Beiprodukt kann, besonders im Fall geringer Mengen, in dem erfindungsgemäß hergestellten Thiazolidin-2-thion verbleiben. Nach einer bevorzugten Ausführungsform wird es jedoch von dem Thiazolidin-2-thion abgetrennt und in mindestens eine der Reaktionszonen zurückgeführt. Das besagte Abtrennen des Beiproduktes erfolgt beispielsweise durch Destillation.

Nach einer weiteren bevorzugten Ausführungsform wird das Beiprodukt nach dem Abtrennen des Thiazolidin-2-thions mit dem Aminoethanol vermischt, das der ersten Reaktionszone kontinuierlich zuläuft.

Es ist überraschend, daß es durch die erfindungsgemäßen Maßnahmen gelingt, in einem kontinuierlichen Prozeß, in dem der Abgasstrom praktisch frei von Schwefelkohlenstoff ist, Thiazolidin-2-thione in praktisch quantitativer Ausbeute, bezogen auf sowohl das eingesetzte Aminoethanol als auch auf Schwefelkohlenstoff, herzustellen. Weiterhin ist es überraschend, daß die Produkte bereits bei einer Verweilzeit der flüssigen Phase in den Reaktionszonen von insgesamt 10—120 min, bevorzugt 10—60 min in guter Ausbeute erhalten werden können.

Die erfindungsgemäß hergestellten Thiazolidin-2-thione, die hierbei flüssig anfallen, können, falls sie bei Raumtemperatur fest sind, ohne weitere Reinigung in bekannter Weise durch Abkühlen, z. B. auf einer Schabewalze, in den handelsüblichen festen Zustand überführt werden.

Nachfolgend wird die Erfindung in Verbindung mit den folgenden Beispielen erläutert, wobei alle Teile und Prozentangaben als Gewichtsteile und Gewichtsprozente anzusehen sind.

## Beispiel 1

Die gegen Wärmeabstrahlung isolierte Reaktionsapparatur besteht aus einem 1-l-Dreihalskolben, auf dessen mittleren Schliff eine Füllkörperkolonne mit Glas-Raschigringen aufgesetzt ist. Auf diese ist ein mit Kühlwasser beschickter Rückflußkühler aufgesteckt. Zwischen Kolonne und Kühler befindet sich ein Zulaufrohr A, das über eine Dosierpumpe mit dem Vorratsgefäß A für das Zulaufgemisch verbunden ist. Durch den zweiten Schliff des Kolbens ragt ein Zulaufrohr B zum Boden des Kolbens, das über eine weitere Dosierpumpe mit dem Vorratsgefäß B für Schwefelkohlenstoff verbunden ist. Durch den dritten Schliff des Kolbens wird der Ablauf so entnommen, daß der Kolben zu ca. 65% konstant gefüllt bleibt. Durch Zulaufrohr A werden kontinuierlich 800 g/h Flüssigkeit eingepumpt, die 407 g/h N-Methylaminoäthanol enthalten, während die Restmenge aus der Rezirkulation stammt. Durch Zulaufrohr B werden kontinuierlich 842 g/h Schwefelkohlenstoff eingepumpt. Aus dem Kolben werden kontinuierlich 1131 g/h Reaktionsgemisch entnommen, dessen Temperatur 161°C beträgt. Das Gemisch wird unter Abkühlung mit Stickstoff durchgelassen, aus dem Abgas werden mit Hilfe einer Tiefkühlfalle bei −75°C 17 g/h Schwefelkohlenstoff auskondensiert, die in das Vorratsgefäß B zurückgegeben werden. Aus dem verbleibenden Reaktionsgemisch werden durch Vakuumdestillation bei 130°C 720 g/h N-Methylthiazolidin-2-thion vom Schmelzpunkt 68−70°C erhalten. Das Sumpfprodukt, 394 g/h, wird zum Vorratsgefäß A rezirkuliert.

Aus dem Rückflußkühler der Apparatur fließt bei Normaldruck kontinuierlich ein Gasgemisch von 25°C ab, das neben Schwefelwasserstoff und Kohlenoxysulfid ca. 0,2 Vol.-% Schwefelkohlenstoff und ca. 0,1 Vol.-% N-Methylaminoäthanol enthält.

Die Ausbeute an N-Methyl-thiazolidin-2-thion, bezogen auf N-Methylaminoäthanol und auf Schwefelkohlenstoff, beträgt einschließlich der Verluste durch das Abgas ca. 99,7%.

## Beispiel 2

Die Reaktionsapparatur besteht aus einer 16bödigen Glockenbodenkolonne. Zwischen dem ersten und zweiten Boden ist ein mit Kühlwasser beschickter Kühler eingeschoben. Ferner ist der Ablauf des 15. Bodens über einen wassergekühlten Kühler mit dem 16. Boden verbunden. Je ein Zulaufstutzen befindet sich am 1. Boden (A), 2. Boden (B), 16. Boden (C) und am Sumpfablaßrohr (D). Temperaturmeßstellen befinden sich auf dem ersten Boden ($T_1$), 5. Boden ($T_5$), 9. Boden ($T_9$), 14. Boden ($T_{14}$) und 16. Boden ($T_{16}$). Die Apparatur wird kontinuierlich mit folgenden Mengen beschickt:

| | |
|---|---|
| Stutzen A: | 349 g/h N-Äthyl-aminoäthanol |
| Stutzen D: | 33 g/h N-Äthyl-aminoäthanol |
| Stutzen B: | 137 g/h Rezirkulationsgemisch |
| Stutzen C: | 653 g/h Schwefelkohlenstoff |

Die Verweilzeit der Flüssigphase über alle Reaktionszonen beträgt ca. 44 min.
Es werden folgende Temperaturen gemessen:

| | |
|---|---|
| $T_1$: | 22°C |
| $T_5$: | 147°C |
| $T_9$: | 163°C |
| $T_{14}$: | 171°C |
| $T_{16}$: | 96°C |

Aus dem kontinuierlich ablaufenden Reaktionsgemisch werden destillativ 630 g/h reines N-Äthyl-thiazolidin-2-thion abgetrennt, während das verbleibende Sumpfprodukt zur Vorratsgefäß für den Zulauf zu Stutzen B rezirkuliert wird.

Das so hergestellte N-Äthyl-thiazolidin-2-thion, ein Öl von bräunlicher Farbe, hat einen Schmelzpunkt von 10,8−11,1°C.

Die Ausbeute, bezogen auf das eingesetzte Aminoäthanol und auf Schwefelkohlenstoff, beträgt 99,8%.

## Beispiel 3

Analog zu Beispiel 1 werden 256 g/h N-tert.-Butylaminoäthanol mit 333 g/h Schwefelkohlenstoff zu 382 g/h N-tert.-Butyl-thiazolidin-2-thion vom Fp. 69−70°C umgesetzt.

Das Reaktionsgemisch enthält nach der Reinigung von Schwefelkohlenstoff nur 2% Beiprodukt und kann ohne destillative Abtrennung des Beiproduktes als Vulkanisationsbeschleuniger verwendet werden.

Beispiel 4

In der in Beispiel 2 beschriebenen Apparatur werden bei einem konstanten Vakuum von 150 mbar die folgenden Mengen kontinuierlich zugeführt:

| | |
|---|---|
| Stutzen A: | 700 g/h N-Cyclohexyl-aminopropanol-2 |
| Stutzen D: | 45 g/h N-Cyclohexyl-aminopropanol-2 |
| Stutzen B: | 630 g/h Rezirkulationsgemisch |
| Stutzen C: | 794 g/h Schwefelkohlenstoff |

Die Verweilzeit liegt bei 21 min.
Man erhält 1044 g/h N-Cyclohexyl-5-methyl-thiazolidin-2-thion vom Schmelzpunkt 86–88° C.

**Patentansprüche**

1. Verfahren zur Herstellung von Thiazolidin-2-thionen durch Umsetzung von 1 Mol eines Aminoethanols mit 2 bis 2,1 Mol Schwefelkohlenstoff bei 20–200° C und 0,1–5 bar Druck, dadurch gekennzeichnet, daß in einem kontinuierlichen Einstufenprozeß in mindestens zwei hintereinandergeschalteten Reaktionszonen die Aminoethanol enthaltende Flüssigkeit in innige Berührung mit der Schwefelkohlenstoff enthaltenden Gasphase gebracht wird, wobei die besagte Flüssigphase kontinuierlich in die erste Reaktionszone eingebracht, in Richtung bis zur letzten Reaktionszone weitergeführt und hinter der letzten Reaktionszone entnommen wird, und wobei die besagte Gasphase kontinuierlich in die letzte Reaktionszone eingebracht, in Richtung bis zur ersten Reaktionszone weitergeführt und hinter der ersten Reaktionszone entnommen wird.

2. Verfahren nach Anspruch 1, dadurch gekennzeichnet, daß die Verweilzeit der Flüssigphase in den besagten Reaktionszonen insgesamt 10–120 min beträgt.

3. Verfahren nach Anspruch 1, dadurch gekennzeichnet, daß die Flüssigphase vor oder hinter der letzten Reaktionszone gekühlt wird.

4. Verfahren nach Anspruch 1, gekennzeichnet durch Abführung eines Teils der Reaktionswärme durch Verdampfen von Aminoethanol und Thiazolidin-2-thion in den Reaktionszonen.

5. Verfahren nach Anspruch 1, dadurch gekennzeichnet, daß hinter der letzten Reaktionszone der in der Flüssigphase noch vorhandene Schwefelkohlenstoff durch Zugabe von Aminoethanol entfernt wird.

6. Verfahren nach Anspruch 1 und 5, dadurch gekennzeichnet, daß aus der entnommenen Flüssigphase das N-substituierte Thiazolidin-2-thion abgetrennt wird und das verbleibende Gemisch der Beiprodukte in mindestens eine Reaktionszone zurückgeführt wird.

7. Verfahren nach Anspruch 1 und 6, dadurch gekennzeichnet, daß das besagte Beiprodukt dem er ersten Reaktionszone zufließenden Aminoethanol zugefügt wird.

8. Verfahren nach Anspruch 1 und 4, dadurch gekennzeichnet, daß die Gasphase vor oder hinter der ersten Reaktionszone gekühlt wird und die auskondensierte Flüssigkeit mindestens einer Reaktionszone zugeführt wird.

**Claims**

1. A process for the production of thiazolidine-2-thiones by the reaction of 1 mol of an amino ethanol with from 2 to 2.1 mol of carbon disulphide at from 20 to 200° C and from 0.1 to 5 bar pressure, characterised in that the liquid phase containing amino ethanol is brought into intimate contact with the gaseous phase containing carbon disulphide in a continuous single stage process in at least two consecutive reaction zones, wherein the liquid phase is introduced continuously into the first reaction zone, conveyed toward the last reaction zone and removed downstream of the last reaction zone and wherein the gaseous phase is introduced continuously into the last reaction zone, conveyed toward the first reaction zone and removed downstream of the first reaction zone.

2. A process according to claim 1, characterised in that the total residence time of the liquid phase in the reaction zones is from 10 to 120 minutes.

3. A process according to claim 1, characterised in that the liquid phase is cooled upstream or downstream of the last reaction zone.

4. A process according to claim 1, characterised by the carrying off of a proportion of the reaction heat by evaporation of amino ethanol and thiazolidine-2-thione in the reaction zones.

5. A process according to claim 1, characterised in that the carbon disulphide still present in the liquid phase is removed downstream of the last reaction zone by the addition of amino ethanol.

6. A process according to claims 1 and 5, characterised in that the N-substituted thiazolidine-2-thione is separated from the liquid phase removed and the remaining mixture of the by-products is recirculated into at least one reaction zone.

7. A process according to claims 1 and 6, characterised in that the by-product is fed to the amino ethanol flowing to the first reaction zone.

8. A process according to claims 1 and 4, characterised in that the gaseous phase is cooled upstream or downstream of the first reaction zone and the condensed liquid is supplied to at least one reaction zone.

**Revendications**

1. Procédé de fabrication de thiazolidine-2-thiones par réaction de 1 mole d'un aminoéthanol avec 2 à 2,1 moles de sulfure de carbone à 20 – 200° C et sous une pression de 0,1 – 5 bars, caractérisé à ce que suivant un procédé continu en un stade, on met en contact intime dans au moins deux zones de réaction raccordées en série la phase liquide contenant l'aminoéthanol avec la phase gazeuse contenant le sulfure de carbone, ladite phase liquide étant introduite continuellement dans la première zone de réaction et étant ensuite dirigée jusqu'à la dernière zone de réaction puis étant prélevée à l'arrière de la dernière zone de réaction, tandis que ladite phase gazeuse est introduite continuellement dans la dernière zone de réaction, est ensuite dirigée jusqu'à la première zone de réaction puis est prélevée en arrière de la première zone de réaction.

2. Procédé selon la revendication 1, caractérisé en ce que le temps de séjour de la phase liquide dans lesdites zones de réaction s'élève en tout à 10 à 120 minutes.

3. Procédé selon la revendication 1, caractérisé en ce que la phase liquide est refroidie avant ou après la dernière zone de réaction.

4. Procédé selon la revendication 1, caractérisé par la dissipation d'une partie de la chaleur de réaction par évaporation de l'aminoéthanol et de la thiazolidine-2-thione dans les zones de réaction.

5. Procédé selon la revendication 1, caractérisé en ce qu'à l'arrière de la dernière zone de réaction le sulfure de carbone encore présent dans la phase liquide est éliminé par une addition d'aminoéthanol.

6. Procédé selon les revendications 1 et 5, caractérisé en ce qu'à partir de la phase liquide prélevée on sépare la thiazolidine-2-thione N-substituée et l'on renvoie le mélange restant des sous-produits dans au moins une zone de réaction.

7. Procédé selon les revendications 1 et 6, caractérisé en ce que ledit sous-produit est ajouté à l'aminoéthanol allant à la première zone de réaction.

8. Procédé selon les revendications 1 et 4, caractérisé en ce que la phase gazeuse est refroidie avant ou après la première zone de réaction et en ce que le liquide qui a été séparé par condensation est fourni au moins à une zone de réaction.